# FASCICULE DE BREVET EUROPEEN

(11) **EP 4 358 986 B1**
(45) Date de publication et mention de la délivrance du brevet: **31.12.2025**
(21) Numéro de dépôt: 22741347.3
(22) Date de dépôt: 17.06.2022
(51) Int. Cl.: A61K 36/30, A61Q 5/12, A61K 8/9789, A61Q 1/10, A61Q 19/00, A61Q 3/00, A61Q 5/00, A61Q 5/02, A61Q 7/00

(54) **UTILISATION COSMETIQUE D'UN EXTRAIT DE PHACELIE**
KOSMETISCHE VERWENDUNG EINES PHYLLIA-EXTRAKTS
COSMETIC USE OF A PHACELIA EXTRACT

(30) Priorité: 21.06.2021 FR 2106581
(43) Date de publication de la demande: 01.05.2024
(73) Titulaire: Laboratoires De Biologie Vegetale Yves Rocher, 56200 La Gacilly (FR)
(72) Inventeur: TOUZEAU, Betty, 92370 CHAVILLE (FR); ZOZINE, Sophie, 91630 MAROLLES EN HUREPOIX (FR); LAPERDRIX, Céline, 78470 SAINT-REMY-LES-CHEVREUSE (FR)
(74) Mandataire: Novagraaf Technologies
(86) Numéro de dépôt international: PCT/FR2022/051179
(87) Numéro de publication internationale: WO 2022/269175

(56) Documents cités:
- WO-A1-2012/129683
- CN-A- 112 569 134
- US-A- 5 824 312
- US-A1- 2011 217 753
- BAJKACZ SYLWIA ET AL: "Determination of Flavonoids and Phenolic Acids in Plant Materials Using SLE-SPE-UHPLC-MS/MS Method", FOOD ANALYTICAL METHODS, SPRINGER NEW YORK LLC, US, vol. 11, no. 12, 6 August 2018 (2018-08-06), pages 3563 - 3575, XP036621751, ISSN: 1936-9751, [retrieved on 20180806], DOI: 10.1007/S12161-018-1332-9
- ANONYMOUS: "Native Plant Fun Facts: Phenomenal Phacelia! - Garden for the Environment", 30 March 2020 (2020-03-30), XP055893519, Retrieved from the Internet <URL:https://www.gardenfortheenvironment.org/growing-gardeners-archive/2020/3/30/native-plant-fun-facts-2-phenomenal-phacelia> [retrieved on 20220218]
- ANONYMOUS: "Phacelia", 13 April 2021 (2021-04-13), XP055893537, Retrieved from the Internet <URL:https://web.archive.org/web/20210413235409/http://www.worldbotanical.com/phacelia.htm> [retrieved on 20220218]

## Description

### Domaine technique

La présente invention se rapporte à une utilisation cosmétique non thérapeutique d'au moins un extrait hydrophile de phacélie appartenant à l'espèce *Phacelia tanacetifolia* choisi parmi un extrait de feuille, de tige, de fleur, de graine, ou l'un de leurs mélanges, comme actif cosmétique anti-âge.

La présente invention trouve une application dans le domaine de la cosmétique et de la dermatologie, plus particulièrement de la cosmétique cutanée.

Dans la description ci-dessous, les références entre crochets ([ ]) renvoient à la liste des références présentée à la fin du texte.

### Etat de la technique

La peau est un organe vital à part entière qui se compose de trois tissus distincts, assumant chacun différents rôles grâce à différents types cellulaires et différentes structures.

Le tissu le plus en surface, et donc le plus exposé, est l'épiderme. Cet épithélium pluristratifié (Malpighien) et kératinisé, se compose de différentes cellules associées à de nombreuses fonctions de barrière et de protection. Les cellules majoritaires sont les kératinocytes, qui se divisent dans la couche basale et entament leur différenciation jusqu'à la couche cornée (couche la plus externe), puis sont éliminés par desquamation, en 21 à 28 jours, en moyenne. Le rôle majeur de l'épiderme est d'apporter à la peau, et donc au corps humain, une première ligne de protection contre les agressions extérieures, comme les agressions physiques, chimiques, hydriques et bactériologiques. Cette protection est notamment assurée par les couches les plus différenciées et la couche cornée, connue pour ses propriétés hydrophobes, son aspect compacte et étanche. Une bonne différenciation et donc une bonne desquamation sont essentielles pour une surface lisse, homogène et régulière. L'ensemble de ces mécanismes dépend directement des capacités prolifératives des kératinocytes. Or, les stress extérieurs et l'âge altèrent les kératinocytes et ralentissent ce processus qui peut alors devenir plus long et plus irrégulier. La desquamation devenant irrégulière et/ou anarchique, des défauts de lissage et d'homogénéité de surface de la peau s'installent.

En position intermédiaire, le derme est un tissu conjonctif investi majoritairement de fibroblastes et de protéines matricielles, donnant à la peau ses qualités de compressibilité et d'élasticité connues. Les modifications de sa texture et de sa composition sont largement responsables des altérations de la peau qui surviennent au cours du vieillissement. Le relief de la couche cornée est, par ailleurs, directement conditionné par la qualité et la densité du derme qui la sous-tend. Plus que l'épiderme, dont le renouvellement est rapide et constant, le derme subit des troubles importants liés à l'âge, par le vieillissement de ses cellules et de sa matière. En vieillissant et suite aux agressions environnementales, les fibroblastes sont de moins en moins réactifs et de moins en moins prolifératifs. Les synthèses des macromolécules de la matrice ne sont plus assurées par les fibroblastes. De plus, ces macro-molécules constitutives du derme telles que les fibres de collagène, les fibres élastiques, les protéoglycanes et les glycosaminoglycanes (acide hyaluronique notamment) ont tendance à dégénérer et à se fragmenter. Ces phénomènes d'altération du tissu dermique provoquent en surface un défaut d'organisation de l'épiderme et, d'une façon plus visible et plus directe, une perte de fermeté pouvant aller jusqu'à la ptose cutanée et/ou la formation de rides.

Au sein de la trame conjonctive, s'intercalent aussi d'autres cellules et structures, tel un important réseau circulatoire et nutritif, constitué des vaisseaux sanguins et des capillaires lymphatiques, ainsi que les annexes épidermiques : cheveux, poils, ongles, glandes pilosébacées et glandes sudoripares, qui prennent naissance dans le derme profond. Comme précédemment avec l'âge et les agressions environnementales, le fonctionnement cellulaire ralentit et les cellules endothéliales constitutives des vaisseaux sont moins jointives et moins fonctionnelles, entraînant des défauts d'irrigation et de nutrition du tégument et de ses annexes (glandes ou cheveux).

L'hypoderme, situé en profondeur et constitué en majeure partie de lobules graisseux (adipocytes), assure une fonction de support primaire, de protection mécanique et thermique, et joue aussi un rôle de stockage des réserves énergétiques rapidement mobilisables pour tous les besoins biologiques, comme par exemple le renouvellement cellulaire, la défense de l'organisme ou la contraction musculaire.

L'aspect de surface de la peau est conditionné par tous ces tissus. Dans l'épiderme, les kératinocytes se divisent (via les mitoses) dans la couche basale et entament leur différenciation jusqu'à la couche cornée puis la desquamation (c'est-à-dire leur élimination par détachement), en 21 à 28 jours, en moyenne. Un bon métabolisme des kératinocytes est essentiel pour une surface lisse, homogène et régulière. Plus en profondeur dans le derme se situe le réseau endothélial qui permet d'irriguer tout l'édifice cutané grâce à la circulation sanguine, apportant eau, nutriments et oxygène. Ce mécanisme dépend directement du bon état physiologique de l'organisme, il permet aussi de donner sa couleur vitale à la peau (teint rosé). Or, les stress extérieurs, le tabac, les UV ralentissent ce processus qui peut alors devenir plus irrégulier et endommagé : tout le réseau composé de cellules endothéliales souffre et s'altère induisant une malnutrition, déshydratation et hypoxie. En complément, la desquamation devient irrégulière et/ou anarchique. Des défauts de lissage et d'homogénéité de surface de la peau, un teint plus hétérogène ou plus jaunâtre, une déshydratation ou une sècheresse s'installent.

Ces mécanismes peuvent arriver de façon consécutive à des stress exogènes ou endogènes, et peuvent être complétement déconnectés de mécanismes liés à l'âge ; en revanche, ces derniers les accentuent.

D'un point de vue général, le processus de vieillissement est un processus généralisé consécutif à l'avancement de l'âge (vieillissement intrinsèque) ou à l'accumulation des effets dus aux agressions environnementales répétées (vieillissement extrinsèque).

Pour le marché cosmétique, il convient donc de stimuler de façon biologique un ou des mécanismes impliqués dans l'aspect esthétique de la peau en lien direct ou indirect avec l'hydratation, la nutrition, l'éclat, la protection de la peau.

Le marché des produits cosmétiques est très important et les attentes des consommateurs/consommatrices sont très élevées. En terme d'anti-âge ou non, ils/elles s'orientent de plus en plus vers des produits efficaces, naturels et éco-conçus. Par exemple, la vitamine C est très connue et a fait ses preuves. Comme les autres vitamines, elle est généralement présente dans les produits anti-âge. Même d'origine naturelle, ce composé est très instable, et nécessite donc l'ajout de conservateurs ou l'utilisation de dérivés non naturels, peu appréciés des consommateurs.

De même, les dérivés d'acide rétinoïque sont connus pour stimuler le renouvellement des kératinocytes, mais fragilisent la fonction barrière issue de la différenciation des kératinocytes et ne jouent en rien sur la détoxification cellulaire, voire même, causent des réactions inflammatoires.

De même, les réponses cosmétiques à la problématique de surface de la peau s'orientent fréquemment vers les alphahydroxyacides, comme les acides glycoliques par exemple, qui présentent une action immédiate due à leur effet desquamant. Toutefois ils peuvent entrainer des dégradations de l'épiderme et amplifier les dégâts induits les UV. De plus, il s'agit souvent d'une action limitée dans le temps qui peut occasionner un effet rebond.

L'utilisation traditionnelle de d'extraits de phacélie (Phacelia californica) pour traiter des affections cutanées (jus de feuilles fraîches pour traiter des maladies de la peau) ainsi que l'utilisation d'extraits aqueux de racines de phacélie sous forme de thé pour traiter la fièvre et les rhumes est décrite dans "Native Plant Fun Facts: Phenomenal Phacelia!-Garden for the Environment", https://www.gardenfortheenvironment.org/growing-gardeners-archive/2020/3/30/native-plant-fun-facts-2-phenomenal-phacelia (2020-03-30). Des espèces de phacélie et des utilisations traditionnelles de leurs extraits dont l'utilisation des racines de P. californica pour la fièvre, le rhume et la toux et l'utilisation des feuilles en dermatologie sont également décrites dans "Phacelia", https://web.archive.org/web/20210413235409/http:// www.worldbotanical.com/phacelia.htm (13.04.2021).

Il reste donc un réel besoin de trouver de nouvelles compositions et/ou composés d'origine naturelle, permettant d'améliorer l'aspect et les qualités de surface de la peau, notamment d'hydratation, de nutrition, de protection, de lissage, de douceur, d'amélioration de l'éclat, de l'éclat et d'homogénéisation du teint de la peau.

### Description de l'invention

La présente invention a précisément pour but de répondre à ces besoins et inconvénients de l'art antérieur.

Les inventeurs sont les tout premiers à utiliser un extrait de phacélie, permettant précisément de répondre efficacement aux besoins précités.

Les inventeurs de la présente ont mis en évidence des propriétés de l'extrait qui n'avaient jamais été décrites ou suggérées dans l'art antérieur. En effet, de manière surprenante, les inventeurs ont mis en évidence que l'extrait de phacélie possède une action cosmétique.

Les inventeurs ont notamment mis en évidence un effet de l'association des extraits de phacélie sur l'esthétisme en général de la peau, sa souplesse, son éclat, sa couleur, son grain, son hydratation, sa nutrition, sa protection et son lissage de surface. Ils ont démontré que l'extrait de phacélie stimulait l'alimentation et l'oxygénation de la peau, tout en améliorant les processus de différenciation kératinocytaire.

Ainsi, un premier objet de l'invention se rapporte à une utilisation cosmétique non thérapeutique d'au moins un extrait hydrophile de phacélie appartenant à l'espèce *Phacelia tanacetifolia* choisi parmi un extrait de feuille, de tige, de fleur, de graine, ou l'un de leurs mélanges, comme actif cosmétique anti-âge.

On entend par « phacélie », au sens de la présente invention, l'espèce *Phacelia tanacetifolia.*

Selon l'invention, l'extrait de phacélie est un extrait de feuille, de tige, de fleur, de graine, de racine, ou l'un de leurs mélanges. S'il s'agit d'un extrait des parties aériennes fleuries, celles-ci peuvent être récoltées en début de floraison (bourgeon), en pleine floraison ou en fin de floraison.

L'extrait hydrophile de phacélie peut être comme défini dans la revendication 3II peut s'agir notamment d'un extrait choisi parmi un extrait alcoolique, par exemple un extrait éthanolique, un extrait aqueux, par exemple un hydrodistillat, un extrait hydro-alcoolique, par exemple séché ou liquide avec reprise ou non dans un solvant cosmétique, ou un extrait glycolique.

L'extrait de phacélie peut être obtenu par tout procédé d'extraction connu de l'homme du métier pour d'autres plantes et adapté à la phacélie. Ainsi, le solvant d'extraction peut être choisi parmi l'eau, les alcools en C1 à C5, par exemple l'éthanol, le méthylpropanediol, le propane-1,3-diol; des glycols en C3 à C5, par exemple le propylène glycol, le butylène glycol, dipropylène glycol; le pentylène glycol, la glycérine l'hexane, l'heptane, l'acétate d'éthyle, le CO₂, les lipides et huiles végétales ; ou leurs mélanges.

Par exemple, le solvant d'extraction peut être l'éthanol, avec une dilution dans un glycol comme la glycérine, le pentylène glycol, le propylène glycol ou le propanediol, ou dans une huile comme l'huile de jojoba, de macadamia et/ou de tournesol.

De façon avantageuse, le solvant d'extraction employé peut être un solvant hydroéthanolique, de préférence dans un mélange eau/éthanol ayant un rapport massique eau/alcool compris entre 90/10 et 10/90, par exemple de l'ordre de 70/30.

L'extrait de phacélie peut être obtenu par tout procédé connu de l'homme du métier, comme, à pression atmosphérique, par macération, infusion, décoction, effleurage, lixiviation, ultra-sons ou micro-ondes, ou sous haute pression avec des solvants à l'état super ou subcritque comme le CO₂ ou l'eau, avec ou sans co-solvant, par entrainement à la vapeur d'eau (hydrodistillation). Par exemple, l'extrait de phacélie peut être obtenu en broyant (ou non) une ou plusieurs parties de la plante, fraiches, congelées, de préférence séchées et coupées ou broyées au préalable, puis en mettant en contact le broyat avec un solvant tel que défini précédemment, préférentiellement un mélange eau/alcool ayant un rapport eau/alcool entre 90/10 et 10/90, par exemple un mélange eau/alcool ayant un rapport massique 70/30. L'extraction peut être conduite en mélangeant le broyat et le solvant et en soumettant ce mélange à une agitation de façon à optimiser les échanges entre le broyat et le solvant d'extraction. L'extraction peut être conduite à une température de 20 à 100°C. Par exemple, l'extraction peut être réalisée pendant une durée de l'ordre de 1/2 à 4 heures. La quantité de plante utilisée par rapport au solvant peut être par exemple de 0,5 à 20 % en poids par rapport au poids total de plantes et de solvant, par exemple 1,0 à 15,0%, ou 3,0 à 12,0% ou 6,0 à 8,0 %, par exemple environ 7%. Il est à noter que l'extrait employé dans la présente invention peut éventuellement être purifié préalablement à sa mise en œuvre dans une composition cosmétique. Cette purification peut par exemple être effectuée par extraction liquide-liquide, par précipitation ou par chromatographie préparative. L'extrait employé dans la présente invention peut être dilué dans un solvant connu de l'homme du métier, comme par exemple le propylène glycol, le butylène glycol, le dipropylène glycol, le pentylène glycol, le méthylpropanediol, le propane-1,3-diol, la glycérine, l'eau ou un mélange de ces solvants. Il peut s'agir par exemple d'un extrait obtenu à partir de parties aériennes fleuries, notamment extraites par un mélange hydroéthanolique et dilué dans la glycérine.

Selon un mode de réalisation, on utilise comme extrait de phacélie, l'extrait brut obtenu qui est issu de l'extraction décrite ci-dessus. **Il** peut donc s'agir du milieu tel qu'il est obtenu directement à l'issue de l'extraction par un des solvants précités, cet extrait brut étant éventuellement filtré préalablement avant son emploi dans une composition cosmétique. Dans ce mode de réalisation, l'extrait employé se présente sous la forme d'une dispersion ou d'une solution dans un milieu liquide incluant le solvant précité.

Selon un autre mode de réalisation, on utilise un extrait séché obtenu en soumettant l'extrait brut précité, de préférence filtré, à une étape ultérieure de séchage, typiquement par atomisation ou par lyophilisation, selon des techniques connues, permettant de préserver l'intégrité des composés présents dans l'extrait. L'extrait obtenu selon ce mode de mise en œuvre se présente généralement sous la forme d'une poudre qui peut être employée telle qu'elle ou reprise dans un solvant ou un dispersant pour formuler une composition cosmétique.

Par exemple, un extrait utilisé dans l'invention est susceptible d'être préparé selon le procédé comprenant les étapes suivantes :
a) Mise en contact de tout ou partie de la plante avec un solvant de type hydroalcoolique, préférentiellement à raison d'au moins 50g/L,
b) Extraction,
c) Séparation des phases soluble et insoluble,
d) Filtrations de l'extrait brute jusqu'à la filtration stérilisante,
e) Eventuellement concentration du produit obtenu à l'étape d),
f) Eventuellement, dilution dans un solvant approprié ou ajout de toute substance permettant de finaliser l'extrait.
g) Eventuellement, pasteurisation et/ou filtration stérilisante de l'extrait obtenu dans les étapes antérieures.

Des étapes de décoloration et désodorisation de la phase soluble peuvent être envisagées sans modifier la fraction active.

L'extrait de phacélie peut être composé d'une fraction phénolique, et peut ainsi comprendre au moins un composé choisi parmi des dérivés d'acides cafféoylquiniques, notamment les 3-CQA, 4-CQA et 5-CQA, des flavonoïdes, notamment la rutine, la naringénine, l'eriodictyol et l' hesperetin, des sucres, notamment le glucose, le fructose, le xylose, le mannose ou le saccharose, des cyclitols avec des dérivés de l'inositol, des acides aminés et protéines, des acides gras, des saponosides, et des composés monoterpéniques, diterpéniques et triterpéniques.

On entend par « composition cosmétique », dans la présente invention, toute composition à visée cosmétique, c'est à dire esthétique, pouvant être mise en contact avec les parties superficielles du corps humain, par exemple l'épiderme, les systèmes pileux et capillaires, les organes externes, les dents et les muqueuses externes. Avantageusement, une composition cosmétique permet, exclusivement ou principalement, de les protéger, parfumer, maintenir en bon état, modifier leur aspect ou en corriger les défauts superficiels. Notamment, une composition cosmétique peut comprendre un véhicule cosmétiquement acceptable.

Dans le cadre de la présente invention, on entend par «composition dermatologique» toute composition à visée dermatologique, c'est à dire une composition pouvant être mise en contact avec les parties superficielles du corps humain, pour un traitement de la peau, des muqueuses et des phanères, par exemple des ongles, des cheveux, et/ou des poils. Notamment, une composition cosmétique peut comprendre un véhicule dermatologiquement acceptable.

Par « véhicule cosmétiquement et/ou dermatologiquement acceptable », on entend un véhicule adapté pour une utilisation en contact avec des cellules humaines cutanées, en particulier les cellules de l'épiderme, sans toxicité, irritation, réponse allergique indue et similaire, et proportionné à un rapport avantage/risque raisonnable. Le véhicule cosmétiquement et/ou dermatologiquement acceptable peut être choisi parmi l'eau, l'allantoïne, la glycérine, le méthylpropanediol, cette liste n'étant pas limitative.

La composition cosmétique ou dermatologique peut être obtenue par tout procédé approprié connu de l'homme du métier pour la fabrication d'une composition cosmétique ou dermatologique. Il peut s'agir, par exemple d'un simple mélange. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 µm.

Dans la composition utilisée dans l'invention, la teneur en extrait de phacélie peut être compris entre 0,01 et 10% du poids total de la composition. Par exemple, la composition peut comprendre de 0,01 à 5 %, ou de 0,01 à 3 %, ou de 0,01 à 2 %, ou de 0,01 à 1 %, ou de 0,1 à 3 %, ou de 0,1 à 1%, ou de 0,05 à 5%, en poids de l'extrait de phacélie par rapport au poids total de la composition.

La composition cosmétique utilisée dans la présente invention peut se trouver sous toute forme appropriée pour une application cosmétique ou dermatologique. Avantageusement, la composition est une composition à usage topique.

Il peut s'agir par exemple d'une composition sous une forme choisie dans le groupe comprenant une émulsion huile dans eau, ou eau dans huile, ou un mélange de ces émulsions.

La composition peut notamment être sous une forme choisie parmi un onguent, une crème, une huile, un lait, une pommade, une poudre, un tampon imbibé, une solution, un gel, un sérum, un baume, un beurre, une lotion, une suspension, un savon ou une émulsion. La composition cosmétique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables pour la mise en œuvre de la présente invention sont connues dans l'état de la technique par les formulateurs. Dans ces exemples de composition, il suffit d'ajouter l'association des huiles essentielles de la présente invention pour obtenir une composition conforme à l'utilisation de la présente invention.

Une composition cosmétique utilisée dans l'invention peut être utilisée seule ou en combinaison avec une ou plusieurs autres substances ou ingrédients actifs ou inactifs cosmétiquement ou dermatologiquement. Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver l'extrait actif dans le temps, etc. Il peut s'agir en outre de tout produit de base pouvant se trouver dans les compositions cosmétiques ou dermatologiques classiques. Par opposition, les substances ou ingrédients actifs sont ceux qui dans l'application cosmétique ou dermatologique visée ont une action esthétique et/ou médicale.

Ainsi, l'extrait de phacélie peut être la seule substance ou ingrédient actif de la composition, ou il peut être associée à d'autres substances ou ingrédients actifs. Avantageusement, la composition utilisée dans l'invention peut notamment comprendre un ou plusieurs agents cosmétiques connus de l'homme du métier, autres que les extraits utilisés dans l'invention.

Un procédé de préparation d'un extrait de phacélie choisi parmi un extrait de feuille, de tige, de fleur, de graine, de racine, ou l'un de leurs mélanges est décrit, le procédé comprenant une étape d'extraction choisie parmi une extraction alcoolique, aqueuse, hydro-alcoolique, glycolique, une macération huileuse et une extraction lipidique. Ces étapes peuvent comprendre les caractéristiques techniques telles que décrit ci-avant.

Le procédé de préparation peut par exemple comprendre une étape d'extraction qui est une extraction hydro-alcoolique, et comprendre en outre :
- mise en contact et extraction de tout ou partie des parties aériennes de phacélie dans un solvant hydroalcoolique,
- séparation des phases soluble et insoluble,
- filtration, concentration du produit, dilution dans un solvant approprié ou ajout de toute substance permettant de finaliser l'extrait de phacélie,
- éventuellement une pasteurisation et/ou filtration stérilisante de l'extrait obtenu dans les étapes antérieures.

Un dispositif se présentant sous une forme choisie parmi un pot, un flacon-pompe, une lingette, un masque, un dispositif transdermique, un patch, un spray, une capsule ou une gélule, le dispositif comprenant une composition ou un extrait utliisés dans l'invention, ou un extrait de phacélie tel que défini ci-avant, est décrit.

Dans l'utilisation cosmétique de l'invention, l'effet anti-âge peut avantageusement provenir d'une action cosmétique d'hydratation, de nutrition et de protection de la peau et/ou de ses annexes et/ou des muqueuses de l'extrait de phacélie.

On entend par « annexes », au sens de la présente invention, les annexes épidermiques qui prennent naissance dans le derme profond, comprenant au moins un élément choisi parmi les poils, les ongles, les glandes pilosébacées et les glandes sudoripares.

On entend par « muqueuse », au sens de la présente invention, notamment les muqueuses externes comme les lèvres.

L'action cosmétique vise notamment à améliorer l'esthétisme de la peau. **Il** s'agit donc d'une action purement esthétique, à l'exclusion de toute action thérapeutique, permettant d'améliorer l'aspect de surface de la peau, et/ou de ses annexes et/ou des muqueuses, notamment pour présenter, à terme, un aspect plus esthétique, régulier et/ou homogène. On entend par « améliorer l'aspect de surface » tout effet permettant d'embellir, d'homogénéiser la partie visible de la peau, et/ou de ses annexes et/ou des muqueuses, et son aspect esthétique associé à des critères de jeunesse. On entend par « critères de jeunesse » tout descriptif lié à la bonne hydratation et/ou la bonne nutrition de la peau, le grain de peau lisse et homogène, sa couleur homogène et vitale, la souplesse et le rebond, le fonctionnement optimum du tissu cutané associé ou pas à une bonne santé et à un état jeune.

Avantageusement, l'action esthétique sus-mentionnée peut être due à une amélioration de la nutrition, de l'hydratation et/ou de l'oxygénation de la peau, notamment par une amélioration de l'angiogenèse et donc du réseau endothélial de la peau. On entend par « la nutrition, de l'hydratation et/ou de l'oxygénation de la peau», au sens de la présente invention, tout effet permettant de stimuler l'apport hydrique, et/ou nutritionnel issu de l'alimentation, et/ou d'oxygène issu de la respiration, pour une utilisation au niveau des cellules de la peau. La circulation sanguine, et donc l'angiogenèse, jouent un rôle important dans ces processus. On entend par « améliorer le réseau endothélial », au sens de la présente invention, tout effet biologique direct ou indirect sur les cellules endothéliales, qui permet de stimuler leur physiologie et/ou leur fonctionnalité, comme par exemple les prolongements cytoplasmiques ou les ramifications cellulaires. On entend par « protection de la peau », au sens de la présente invention, tout effet permettant de stimuler la fonction barrière protectrice de la surface de l'épiderme, notamment lié au processus de différenciation kératinocytaire.

Avantageusement, l'action cosmétique peut être tout processus lié au vieillissement intrinsèque ou extrinsèque pouvant avoir lieu au niveau de la cellule sans pour autant être systématiquement visible au niveau de l'organe.

Avantageusement, cette association permet en outre d'apporter un effet cosmétique sur la surface de la peau, notamment choisi parmi l'amélioration de la finesse du grain de peau, l'amélioration de la couleur de la peau, l'amélioration du lissage de la peau, l'amélioration de la douceur de la peau, annexes et/ou muqueuses, l'amélioration de l'éclat de la peau, l'amélioration des propriétés biomécaniques de la peau, l'amélioration de la souplesse de la peau, la lutte et la prévention des effets visibles du vieillissement de la peau, annexes et/ou muqueuses notamment le relâchement et/ou la perte de fermeté et/ou la perte d'élasticité et/ou la formation des rides, de préférence avant que les effets du vieillissement ne soient visibles.

Ainsi, l'association permet d'obtenir un effet général esthétique sur l'aspect de surface et la beauté de la peau. Sans vouloir être lié par un mécanisme biologique particulier, cet effet serait dû en tout ou partie à un effet de l'extrait choisi parmi l'amélioration de la nutrition, l'amélioration de l'hydratation, l'amélioration de l'oxygénation et l'amélioration de la protection de la peau, de ses annexes et/ou de ses muqueuses.

Un procédé de soin cosmétique non thérapeutique pour améliorer l'aspect de la peau, comprenant une application sur la peau ou les muqueuses d'une composition cosmétique décrit précédemment, est décrit.

Avantageusement, le soin cosmétique est choisi parmi l'hydratation, la nutrition, l'oxygénation et la stimulation de la fonction barrière protectrice de la peau et/ou de ses annexes et/ou des muqueuses.

Avantageusement, le procédé cosmétique non thérapeutique est un procédé de soin esthétique de la surface de la peau, de ses annexes et/ou des muqueuses, pour une action sur l'esthétisme et le vieillissement de la peau et/ou de ses annexes et/ou des muqueuses, notamment pour apporter éclat, souplesse, finesse, lissage et/ou douceur. Avantageusement, le procédé de soin permet d'améliorer l'aspect esthétique de la peau.

Dans le cadre des procédés cosmétiques décrits, l'utilisation s'entend d'une utilisation non-thérapeutique, par exemple pour le traitement des peaux normales. Dans la présente, on entend par « peaux normales », des peaux ne présentant ou n'étant pas susceptible de présenter un état pathologique.

D'une manière générale, toute utilisation cosmétique et tout procédé cosmétique sont respectivement des utilisations cosmétiques non-thérapeutiques et procédés cosmétiques non-thérapeutiques.

D'autres avantages pourront encore apparaître à l'homme du métier à la lecture des exemples ci-dessous, donnés à titre illustratif.

### Brève description des figures

[Fig. 1] représente l'intensité de la filaggrine par plan (couches du micro-épiderme) pour le contrôle (trait plein), le micro-épiderme (ME) traité avec un extrait de phacélie à 10⁻⁴% (tirets) et le ME traité avec un extrait de phacélie à 10⁻³% (pointillés).
[Fig. 2] représente l'expression de filaggrine totale (en pourcentage par rapport au témoin) pour le contrôle, le micro-épiderme (ME) traité avec un extrait de phacélie à 10⁻⁴% et le ME traité avec un extrait de phacélie à 10⁻³%.

### EXEMPLES

### Exemple 1 : Préparation de l'extrait de phacélie

Un extrait utilisé dans l'invention est préparé selon le procédé comprenant les étapes suivantes :
- mise en contact des parties aériennes fleuries de phacélie, broyées, avec un solvant hydroéthanolique, c'est-à-dire avec un mélange eau/ éthanol ayant un rapport massique 70/30. La quantité de plante utilisée par rapport au solvant est de 7% en poids par rapport au poids total de plantes et de solvant,
- extraction pendant une durée de l'ordre de 1H30 min à 50°C,
- filtration pour la séparation du résidu sec et du jus d'extraction et jusqu'à une filtration stérilisante,
- concentration sous vide de l'extrait filtré,
- ajout de glycérine pour standardiser l'extrait à une masse sèche de 2%
- pasteurisation et/ou filtration stérilisante.

### Exemple 2 : Effet de l'extrait de phacélie sur les mécanismes de l'angiogenèse

L'angiogenèse est un terme biologique qui regroupe de nombreux processus reliés au développement du réseau sanguin (artères, vaisseaux, capillaires...). Dans le derme, les capillaires sanguins se forment par l'agencement tridimensionnel des cellules endothéliales. Ces cellules sont particulièrement jointives et développent de nombreuses ramifications pour assurer le réseau qui irrigue, alimente et oxygène tout le tissu cutané. Favoriser ces interactions est un mécanisme angiogène.

Dans cette expérience, des cellules endothéliales transduites (GFP-HUVEC) pour mise en évidence du cytoplasme par marquage fluorescent, sont cultivées avec des fibroblastes humains normaux dans un mélange de milieu Endothelial Cell Growth 2 supplémenté avec hEGF, hbFGF, R3 IGF, hydrocortisone, vitamine C, et de milieu DMEM supplémenté avec 2 mM L-glutamine, 50 U/ml pénicilline, 50µg/ml streptomycine et 1% de sérum de veau fœtal au ratio 50/50. Les cellules en co-culture sont traitées, ou non, avec le VEGF à 100 ng/ml (Sigma, réf. V7259), ou encore avec l'extrait de phacélie à 10-3% et 10-4% (p/v). Les traitements ont lieu à J1 et J4 en milieu faible en sérum. Les points de branchement sont comptés pour évaluer les capacités des cellules endothéliales à former leur réseau en temps réel grade à Incucyte SIII. Les pourcentages sont calculés en fonction du contrôle milieu. Le VEGF (référence positive) valide les manipulations.

**[Table 1]**

| Actif | Points de branchements |
|---|---|
| Phacélie 10⁻³% | +55% |
| Phacélie 10⁻⁴% | +23% |
| VEGF 50ng/mL | +473% |

L'extrait de phacélie présente un effet stimulant sur le processus d'angiogenèse par augmentation du nombre de branchements inter-cellulaires. A savoir, pour la concentration la plus faible, +55% de points branchements entre cellules, +23»% à la plus forte.

L'extrait de phacélie permet donc stimuler un paramètre associé à l'angiogenèse, et permet donc de mieux nourrir, hydrater et oxygéner le tissu cutané pour des effets esthétiques visibles.

### Exemple 3 : Effet de l'extrait de phacélie sur les mécanismes de différenciation kératinocytaire

La différenciation des kératinocytes est le processus qui permet l'édification de la fonction barrière à la surface de l'épiderme constituée de kératinocytes cornifiés. Durant ce processus, ils perdent leur capacité de prolifération et évoluent biochimiquement en exprimant des kératines particulières ou des marqueurs plus tardifs comme la filaggrine. Cette différenciation est accessible en culture monocouche mais reste imparfaite, c'est pourquoi l'utilisation de modèle tridimensionnel est plus pertinent scientifiquement pour mesurer un effet sur ce processus.

Les tests ont été réalisés sur des micro-épidermes Episcreen^{™} (société Cytoo) reconstruits à partir de kératinocytes humains néonataux en P6, ensemencés sur plaques coatées au collagène I.

Des études préliminaires sont réalisées afin de pouvoir valider le modèle et l'essai, utilisant 2 témoins positifs : la trichostatine A (TSA) et l'acide all-trans rétinoïque (ATRA) ; suivant 3 paramètres : la structure des micro-épidermes, le nombre de noyaux par micro-épiderme, les expressions des marqueurs Ki-67 et filaggrine et 2 points de cinétique, J2 et J4.

Les micro-épidermes différencient normalement en condition contrôle, atteignant le niveau d'expression attendu de filaggrine.

ATRA stimule la prolifération des kératinocytes induisant une augmentation significative du nombre de noyaux dans la couche basale. Comme les micro-épidermes se différencient fortement et rapidement, le pourcentage de Ki-67 exprimé par les cellules est faible et variable pour la condition contrôle, mais tend à augmenter après traitement avec le témoin ATRA.

TSA stimule la différenciation des micro-épidermes, ce qui se traduit par une augmentation significative de l'expression de la filaggrine.

Le traitement avec l'extrait de phacélie 10⁻³% et 10⁻⁴% (p/v) ne présente pas d'impact majeur sur le nombre de noyaux comptés par micro-épiderme.

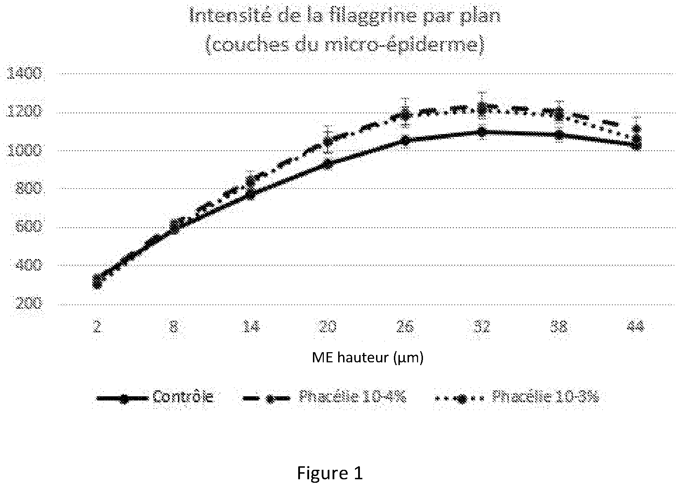

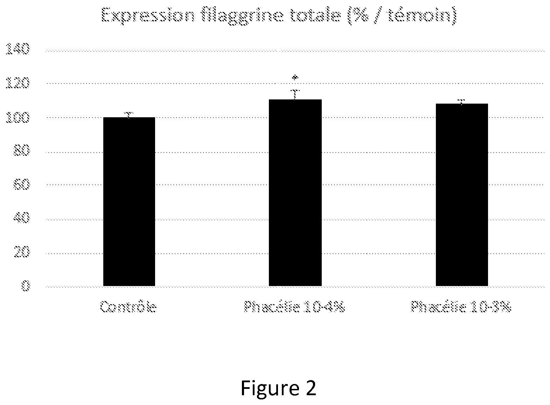

Globalement, les expressions de Ki-67 sont très faibles et peu variables. L'intensité de la filaggrine a été mesurée sur 8 plans z (voir Figure 1) et normalisée à la condition de contrôle (voir Figure 2).

L'extrait de phacélie présente des propriétés stimulant la différenciation des kératinocytes. En effet, l'expression de la filaggrine par les cellules est augmentée en présence de cet extrait, et notamment de manière significative (+11%) à la concentration 10⁻⁴% (p/v).

Sur les différents plans du micro-épiderme, l'effet de l'extrait de phacélie est le plus important entre 14µm et 38µm d'épaisseur, soit les couches les plus hautes, donc les plus différenciées.

L'extrait de phacélie stimule donc l'expression de la filaggrine, la différenciation kératinocytaire et de ce fait la fonction barrière protectrice de l'épiderme.

### Exemple 4 : exemple de composition de soin du visage de type Crème

Les pourcentages sont donnés en masse par rapport à la masse totale de la composition.

**[Table 2]**

| | |
|---|---|
| Eau | Q.S.P. 100% |
| Agent(s) texturant(s) | 0,05 à 0,5% |
| Glycol(s) | 0,5 à 10% |
| Glycérine | 0,5 à 10% |
| Montanov^{™} L | 1 à 7% |
| Alcool(s) gras | 0,5 à 8% |
| Acide(s) gras | 0,5 à 3% |
| Ester(s) gras | 0,5 à 5% |
| Glyceryl stearate & PEG-100 Stearate | 1 à 5% |
| Huile(s) végétales | 0,5 à 5% |
| Beurre(s) végétal(aux) | 0,5 à 5% |
| Polybutène | 0,5 à 5% |
| Cegesoft^{™} | 0,5 à 5% |
| Diméthicone | 0,5 à 5% |
| Lubrajel^{™} MS | 1 à 7% |
| Sodium ascorbyl phosphate | 0,2 à 2% |
| Extrait de PHACELIE | 0,01 à 2% |
| Autre(s) huile(s) essentielle(s) | 0,01 à 4% |
| Autre(s) extrait(s) de plante(s) | 0,5 à 10% |
| Eau(x) florale(s) | 1 à 20% |
| Antioxydant(s) | 0,01 à 1% |
| Parfum | Q .S. |
| Agent(s) de conservation | Q.S. |
| Agent(s) régulateur(s) de pH | 0,01 à 1% |

### Exemple 5 : exemple de composition de soin contour des yeux

Les pourcentages sont donnés en masse par rapport à la masse totale de la composition.

**[Table 3]**

| | |
|---|---|
| Eau | Q.S.P. 100% |
| Agent(s) texturant(s) | 0,05 à 0.5% |
| Sodium polyacrylate | 0.5 à 5% |
| Glycol(s) | 0,5 à 10% |
| Glycérine | 0,5 à 10% |
| Ceteareth-2 phosphate | 0,5 à 5% |
| Sorbitan stearate | 0,5 à 5% |
| Alcool(s) gras | 0,5 à 5% |
| Ester(s) gras | 5 à 10% |
| Huile(s) végétale(s) | 0,5 à 10% |
| Caféine | 0,05 à 1% |
| Escine | 0,2 à 1% |
| Biosaccharide Gum-4 | 0,5 à 5% |
| Extrait de PHACELIE | 0,01 à 2% |
| Autre(s) huile(s) essentielle(s) | 0,01 à 4% |
| Autre(s) extrait(s) de plante(s) | 0,5 à 10% |
| Eau(x) florale(s) | 1 à 10% |
| Vitamine(s) | 0,2 à 2% |
| Pigment(s) optique(s) | 0,3 à 5% |
| Poudre(s) absorbante(s) | 1 à 5% |
| Antioxydant(s) | 0,01 à 1% |
| Parfum | QS. |
| Agent(s) de conservation | QS. |
| Agent(s) régulateur(s) de pH | 0,01 à 1% |

### Exemple 6 : exemple de composition de soin du visage de type sérum

Les pourcentages sont donnés en masse par rapport à la masse totale de la composition.

**[Table 4]**

| | |
|---|---|
| Eau | Q.S.P. 100% |
| Agent(s) Texturant(s) | 0,05 à 0,5% |
| Glycol(s) | 0,5 à 10% |
| Glycérine | 0,5 à 10% |
| Sodium stearoyl glutamate | 0,5 à 5% |
| Ceteth-20 | 0,5 à 5% |
| Sorbitan stearate | 1 à 5% |
| Ester(s) gras | 1 à 10% |
| Diméthicone | 0,5 à 5% |
| Lubrajel^{™} MS | 1 à 5% |
| Alcool | 3 à 10% |
| Extrait de PHACELIE | 0,01 à 2% |
| Autre(s) huile(s) essentielle(s) | 0,01 à 4% |
| Autre(s) extrait(s) de plante(s) | 0,5 à 10% |
| Eau(x) florale(s) | 1 à 20% |
| Antioxydant(s) | 0,01 à 1% |
| Parfum | QS. |
| Agent(s) de conservation | QS. |
| Agent(s) régulateur(s) de pH | 0,01 à 1% |

### Exemple 7 : exemple de composition de soin de protection solaire

Les pourcentages sont donnés en masse par rapport à la masse totale de la composition.

**[Table 5]**

| | |
|---|---|
| Eau | Q.S.P. 100% |
| Agent Texturant | 0,05 à 0,5% |
| Glycol(s) | 0,5 à 10% |
| Gomme xanthane | 0,05 à 0.5% |
| Eumulgin^{™} VL75 | 1 à 7% |
| Stéareth-2 | 1 à 7% |
| Octyl méthoxycinnamate | 3 à 10% |
| Tinosorb M | 3 à 10 % |
| Tinosorb S | 3 à 10% |
| Ester(s) gras | 1 à 15% |
| Diméthicone | 0,5 à 5% |
| Extrait de PHACELIE | 0,01 à 2% |
| Autre(s) huile(s) essentielle(s) | 0,01 à 4% |
| Autre(s) extrait(s) de plante(s) | 0,5 à 10% |
| Eau(x) florale(s) | 1 à 20% |
| Antioxydant(s) | 0,01 à 1% |
| Poudre(s) absorbante(s) | 1 à 5% |
| Parfum | QS. |
| Agent(s) de conservation | QS. |
| Agent(s) régulateur(s) de pH | 0,01 à 1% |

## Revendications

1. Utilisation cosmétique non thérapeutique d'au moins un extrait hydrophile de phacélie appartenant à l'espèce *Phacelia tanacetifolia* choisi parmi un extrait de feuille, de tige, de fleur, de graine, ou l'un de leurs mélanges, comme actif cosmétique anti-âge.

2. Utilisation selon la revendication 1, dans laquelle ledit extrait est un extrait des parties aériennes fleuries.

3. Utilisation selon la revendication 1 ou 2, dans laquelle ledit au moins un extrait hydrophile est choisi parmi un extrait alcoolique, un extrait aqueux, un extrait hydro-alcoolique, un extrait glycolique, et un extrait obtenu sous haute pression avec des solvants à l'état super ou subcritque, par entraînement à la vapeur d'eau.

4. Utilisation selon la revendication 3, dans laquelle ledit extrait est un extrait hydro-alcoolique.

5. Utilisation selon l'une quelconque des revendications précédentes, ledit au moins un extrait étant dans une composition cosmétique comprenant de 0,01 et 10% en poids dudit extrait par rapport au poids total de la composition et au moins un véhicule cosmétiquement acceptable.

6. Utilisation selon la revendication 5, ladite composition étant sous une forme choisie parmi un onguent, une crème, une huile, un lait, une pommade, une poudre, un tampon imbibé, une solution, un gel, un sérum, un baume, un beurre, une lotion, une suspension, un savon et une émulsion.

## Patentansprüche

1. Nicht-therapeutische kosmetische Verwendung mindestens eines hydrophilen Extrakts aus Phacelia der Art *Phacelia tanacetifolia,* ausgewählt aus einem Extrakt aus Blättern, Stängeln, Blüten, Samen oder einer Mischung davon, als Anti-Aging-Kosmetikwirkstoff.

2. Verwendung gemäß Anspruch 1, wobei der Extrakt ein Extrakt aus blühenden oberirdischen Teilen ist.

3. Verwendung gemäß Anspruch 1 oder 2, wobei der mindestens eine hydrophile Extrakt ausgewählt ist aus einem alkoholischen Extrakt, einem wässrigen Extrakt, einem hydroalkoholischen Extrakt, einem Glykolextrakt und einem Extrakt, der unter hohem Druck mit überkritischen oder unterkritischen Lösungsmitteln oder durch Wasserdampfabtrieb gewonnen wurde.

4. Verwendung gemäß Anspruch 3, wobei der Extrakt ein hydroalkoholischer Extrakt ist.

5. Verwendung gemäß einem der vorstehenden Ansprüche, wobei der mindestens eine Extrakt in einer kosmetischen Zusammensetzung enthalten ist, die 0,01 bis 10 Gew.-% des Extrakts, bezogen auf das Gesamtgewicht der Zusammensetzung, und mindestens einen kosmetisch akzeptablen Träger enthält.

6. Verwendung gemäß Anspruch 5, wobei die Zusammensetzung in einer Form vorliegt, die aus einer Salbe, Creme, Öl, Milch, Pommade, Pulver, getränktem Pad, Lösung, Gel, Serum, Balsam, Butter, Lotion, Suspension, Seife und Emulsion ausgewählt ist.

## Claims

1. Non-therapeutic cosmetic use of at least one hydrophilic extract of phacelia belonging to the species *Phacelia tanacetifolia,* chosen from among an extract of leaf, stem, flower, seed, or a mixture thereof, as an anti-ageing cosmetic active ingredient.

2. Use according to claim 1, wherein said extract is an extract from flowering aerial parts.

3. Use according to claim 1 or 2, wherein said at least one hydrophilic extract is selected from an alcoholic extract, an aqueous extract, a hydroalcoholic extract, a glycolic extract, and an extract obtained under high pressure with supercritical or subcritical solvents by steam distillation.

4. Use according to claim 3, wherein said extract is a hydroalcoholic extract.

5. Use according to any of the preceding claims, said at least one extract being in a cosmetic composition comprising 0.01 to 10% by weight of said extract relative to the total weight of the composition and at least one cosmetically acceptable vehicle.

6. Use according to claim 5, wherein said composition is in a form selected from an ointment, cream, oil, milk, salve, powder, soaked pad, solution, gel, serum, balm, butter, lotion, suspension, soap and emulsion.
